# EUROPÄISCHE PATENTSCHRIFT

(11) **EP 0 582 239 B1**
(45) Veröffentlichungstag und Bekanntmachung des Hinweises auf die Patenterteilung: **04.08.1999**
(21) Anmeldenummer: 93112323.6
(22) Anmeldetag: 31.07.1993
(51) Int. Cl.: A61K 9/127, A61K 31/20, A61K 31/685, A61K 7/00

(54) **Pharmazeutische und/oder kosmetische Zubereitung**
Pharmaceutical and/or cosmetic preparation
Préparation pharmaceutique et/ou cosmétique

(30) Priorität: 04.08.1992 DE 4225697; 10.07.1993 DE 4323174
(43) Veröffentlichungstag der Anmeldung: 09.02.1994
(73) Patentinhaber: RHONE-POULENC RORER GMBH, 50792 Köln (DE)
(72) Erfinder: Ghyczy, Miklos, Dr., D-50993 Köln (DE); Gareiss, Johannes, D-50767 Köln (DE); Hager, Jörg-Christian, D-50827 Köln (DE); Wendel, Armin, D-50859 Köln (DE); Nissen-Zoufal, Brigitte, D-53347 Alfter (DE)
(74) Vertreter: Lau-Loskill, Philipp, Dipl.-Phys.

(56) Entgegenhaltungen:
- EP-A- 0 147 741
- EP-A- 0 331 489
- DE-A- 4 021 082
- FR-A- 2 649 322
- US-A- 4 830 858
- SEIFEN- öLE-FETTE-WACHSE Bd. 117, Nr. 10 , 26. Juni 1991 , AUGSBURG (DE) Seiten 372 - 378 XP228115 J. RÖDING ET AL. 'beeinflussung der hautfeuchtigkeit durch liposomen, stabilisierung von pflegenden ölen und lipophilen wirkstoffen mit liposomen'
- SEIFEN-öLE-FETTE -WACHSE Bd. 114, Nr. 14 , 1. September 1988 , AUGSBURG (DE) Seiten 531 - 534 XP24821 H. LAUTENSCHLÄGER ET AL. 'über die verwendung von liposomen aus soja-phospholipiden in der kosmetik'

## Beschreibung

Die vorliegende Erfindung betrifft eine pharmazeutische und/oder kosmetische Zubereitung mit den Merkmalen des Oberbegriffs des Patentanspruches 1

Pharmazeutische und/oder kosmetische Zubereitungen zur topischen Anwendung sind seit langem bekannt. In den üblichen Salben, Cremes, Lösungen, Emulsionen oder Suspensionen, die in der Regel rein örtlich auf der Oberfläche des behandelten Hautbereiches wirken, sind auch solche Systeme bekannt, die ein Trägermaterial aufweisen, wobei das Trägermaterial sicherstellen soll, daß der in der Zubereitung enthaltende Wirkstoff in die Haut bzw. durch die Hautbarriere transportiert wird.

So beschreibt beispielsweise die DE 40 21 082 A eine Reihe von kosmetischen Zubereitungen, die als fünf Hauptbestandteile jeweils eine Bilayer-Quelle, ein Lipid bzw. ein Lipidgemisch, Salze organischer Säuren, Alkohol und einen Stabilisator aufweisen. Konkret ist dann in einem Ausführungsbeispiel dieser Entgegenhaltung eine Anti-Akne-Milch beschrieben, die eine einheitliche und homogene Mischung darstellt, wobei die Anti-Akne-Milch als anwendungsfertige Mischung dem jeweiligen Anwender zur Verfügung gestellt wird. Die in der bekannten Anti-Akne-Milch enthaltenen Stabilisatoren, so insbesondere Harnstoff und/oder Sorbitol, können jedoch zu entsprechenden Nebenwirkungen, wie beispielsweise Hautreizungen und/oder Entzündungen, führen, wobei die Gefahr des Auftretens derartiger Nebenwirkungen insbesondere bei Akne-Patienten gegeben ist.

Eine pharmazeutische und/oder kosmetische Zubereitung mit den Merkmalen des Oberbegriffs des Patentanspruchs 1 ist aus der DE 38 29 899 A bekannt. Hierbei weist die bekannte Zubereitung als Wirkstoff Phospholipidderivate der nachfolgenden allgemeinen Formel (I) auf.

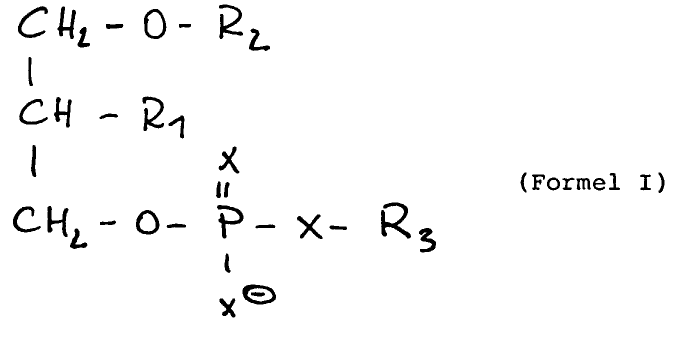

In der Formel I bedeuten R₁ Wasserstoff eine niedrige aliphatische Alkylgruppe mit 1 bis 12 Kohlenstoffatomen, eine Alkoxygruppe mit etwa 6 bis 30 Kohlenstoffatomen, eine Alkylcarbonyloxidgruppe mit etwa 6 bis 30 Kohlenstoffatomen, ein Furanosyl- oder Pyranosylrest mit 4 bis etwa 50 Kohlenstoffatomen und insgesamt bis zu 10 glykosidisch verknüpfte Furanose- und/oder Pyranoseringen, R₂ eine aliphatische Alkylgruppe mit etwa 6 bis 30 Kohlenstoffatomen oder eine aliphatische Alkylcarbonylgruppe mit etwa 6 bis 30 Kohlenstoffatomen, X Sauerstoff, Schwefel oder die Iminogruppe, R₃ der Rest eines Aminoalkohols der allgemeinen Formel R₄ - N (R₅ R₆), wobei R₄ eine ggf einen Carboxylrest tragende Alkylen-Brückengruppe mit 1 bis etwa 12 Kohlenstoffatomen, R₅ und R₆ unabhängig voneinander Wasserstoff oder eine niedrige Alkylgruppe mit einem bis 4 Kohlenstoffatomen sind.

Bezüglich der Dosierformen führt die DE 38 29 899 A lediglich aus, daß die vorstehend durch die Formel I wiedergegebenen Wirkstoffe in den üblichen Dosierformen, so insbesondere als Lösung, Lotion, Salben, Cremes, Gele, Sprays und/oder Aerosole, zur topischen Anwendung gelangen. Ausdrücklich stellt die DE 38 29 899 A jedoch heraus, daß die bekannte Zusammensetzung nur dann bei Akne oder Psoriasis zu einem Behandlungserfolg führt, wenn die Behandlung mit der bekannten Zusammensetzung mit einem Aufenthalt am Toten Meer überlagert wird. Hieraus ist zu schließen, daß die alleinige Anwendung der bekannten Zusammensetzung nur eine beschränkte Wirksamkeit besitzt.

Der vorliegenden Erfindung liegt die Aufgabe zugrunde, eine pharmazeutische und/oder kosmetische Zubereitung zur topischen Anwendung zur Verfügung zu stellen, die eine besonders hohe pharmazeutische und/oder kosmetische Wirksamkeit bei der Prophylaxe und/oder Therapie von Akne und/oder der mit Akne verbundenen Begleiterkrankungen der Haut und/oder bei unreiner Haut besitzt.

Diese Aufgabe wird erfindungsgemäß durch eine pharmazeutische und/oder kosmetische Zubereitung mit den kennzeichnenden Merkmalen des Patentanspruchs 1 gelöst.

Die erfindungsgemäße pharmazeutische und/oder kosmetische Zubereitung, die topisch zur Prophylaxe und/oder Therapie von Akne und/oder der mit Akne verbundenen Begleiterkrankungen der Haut und/oder unreiner Haut angewendet wird, enthält mindestens ein phospholipidisches Trägermaterial sowie mindestens einen Wirkstoff, wobei die gelartige oder flüssige Zubereitung einen solchen Wirkstoff aufweist, der chemisch derart an das phospholipidische Trägermaterial gebunden ist, daß das mit Linolsäure acylierte phospholipidische Trägermaterial ein 1,2-Diacylglycero-3-Phosphatidylcholin ist. Hierbei umfaßt die erfindungsgemäße Zubereitung eine erste Komponente und eine hiervon getrennt verpackte zweite Komponente, wobei die erste Komponente ein Lösungsmittel, mindestens einen Elektrolyten und/oder Wasser und die zweite Komponente das mit Linolsäure acylierte phospholipidische Trägermaterial sowie ggf. Lösungsmittel enthält. Die erste und die zweite Komponente sind zur Erstellung einer anwendungsfertigen Zubereitung miteinander mischbar. Mit anderen Worten umfaßt die erfindungsgemäße Zubereitung zwei getrennt verpackte Komponenten, wobei diese beiden getrennt voneinander verpackten Komponenten erst unmittelbar vor der Anwendung miteinander vermischt werden.

Ein wesentlicher Vorteil der erfindungsgemäßen Zusammensetzung ist darin zu sehen, daß die erfindungsgemäße Zusammensetzung auch ohne Zusatz von Stabilisatoren eine extrem große Lagerstabilität besitzt. Ein derartiger Verzicht auf entsprechende Stabilisatoren bei der erfindungsgemäßen Zusammensetzung führt desweiteren dazu, daß entsprechende Nebenwirkungen, wie beispielsweise Hautreizungen und -entzündungen, die insbesondere bei Akne-Patienten sehr leicht auftreten und teilweise durch die in den bekannten Zubereitungen enthaltenen Stabilisatoren, insbesondere durch Harnstoff, hervorgerufen werden, bei der erfindungsgemäßen Zubereitung total vermieden werden.

Auch weist die erfindungsgemäße pharmazeutische und/oder kosmetische Zubereitung noch eine Reihe von weiteren Vorteilen auf. So ist festzuhalten, daß eine derartige Zubereitung eine besonders hohe pharmazeutische und/oder kosmetische Wirksamkeit, insbesondere auch bei Hauterkrankungen und vorzugsweise bei unreiner Haut, Akne und den damit verbundenen Begleiterscheinungen, besitzt. Bedingt dadurch, daß die erfindungsgemäße Zubereitung topisch, d.h. somit über die Haut, appliziert wird, sind die von der erfindungsgemäßen pharmazeutischen und/oder kosmetischen Zusammensetzung hervorgerufenen Nebenwirkungen minimalisiert, was für solche bekannten Produkte nicht zutrifft, die beispielsweise oral oder durch Injektion appliziert werden. Auch zeigte sich überraschend, daß bereits nach wenigen topischen Anwendungen der erfindungsgemäßen Zubereitung die auf der unreinen Haut vorhandenen Pickel, Quaddeln, Pusteln, Entzündungen oder Mitessern deutlich zurückgingen, so daß bereits nach wenigen Tagen der Anwendung der erfindungsgemäßen Zubereitung eine reine, geschmeidige und glatte Haut resultierte. Auch bei Akne, insbesondere bei schwierigen und langanhaltenden Akne-Erkrankungen, ließ sich durch Anwendung der erfindungsgemäßen Zubereitung innerhalb von relativ kurzen Behandlungszeiträumen, die abhängig von dem Erkrankungsgrad zwischen 2 Wochen und etwa 6 Wochen variierten, eine deutliche Besserung und in den überwiegenden Fällen eine anhaltende Heilung, erzielen, was im Vergleich mit herkömmlichen Akne-Mitteln nicht möglich war. Darüber hinaus wurde die mit der erfindungsgemäßen pharmazeutischen und/oder kosmetischen Zubereitung behandelten Hautbereiche durch die Behandlung deutlich geschmeidiger und elastischer, was bei den behandelten Personen ein Eindruck von gesunder Haut hervorrief.

Wie bereits vorstehend ausgeführt ist, enthält die erste Komponente der erfindungsgemäßen Zubereitung ein Lösungsmittel, mindestens einen Elektrolyten und/oder Wasser, wobei es sich bei dem Lösungsmittel um die nachfolgend noch genannten Lösungsmittel handelt. Als Elektrolyten kann jeder, im medizinischer Bereich eingesetzter Elektrolyt eingesetzt werden, vorzugsweise jedoch handelt es sich bei dem Elektrolyten um eine physiologische Kochsalzlösung. Die zweite Komponente, die, wie bereits vorstehend erwähnt ist, von der ersten Komponente getrennt verpackt ist enthält das mit linolsäure acylierte phospholipidische Trägermaterial sowie ggf. ein Lösungsmittel der nachstehend genannten Art. Der Zusatz des Lösungsmittels zur zweiten Komponente weist den Vorteil auf, daß in der erfindungsgemäßen Zubereitung zwei flüssige Komponenten vorliegen, die unmittelbar vor der Anwendung sehr leicht miteinander vermischt werden können.

Um bei der topischen Anwendung der erfindungsgemäßen Zubereitung die Aufnahme der Zubereitung durch die Haut zu erleichtern, besitzt die erfindungsgemäße Zubereitung vorzugsweise eine flüssige bis halbfeste Konsistenz d. h. die erfindungsgemäße Zubereitung ist insbesondere als Gel oder Flüssigkeit formuliert. Eine derartige gelartige bzw. flüssige Formulierung der erfindungsgemäßen Zubereitung wird insbesondere dadurch erreicht, daß die erfindungsgemäße Zubereitung neben dem Wirkstoff Wasser und/oder ein nicht toxisches Lösungsmittel, insbesondere einen wasserlöslichen Alkohol, aufweist. Hierfür eignen sich insbesondere als wasserlöslicher Alkohol Ethanol, Propanol-1 und Propanol-2, und/oder Propylenglykol. Unter den Begriff Wasser fallen im Rahmen der vorliegenden Beschreibung alle wäßrigen Systeme, wie insbesondere gereinigtes Wasser, destilliertes Wasser, entioniertes Wasser sowie wäßrige Salzlösungen, vorzugsweise physiologische Kochsalzlösungen, oder Puffersysteme, vorzugsweise Phosphatpuffer.

Eine erste Ausbildung der erfindungsgemäßen Zubereitung sieht vor , daß diese ein mit linolsäure acyliertes Phospholipid bzw. ein Phospholipidgemisch umfaßt. Hierunter fallen insbesondere die aus pflanzlichen und/oder tierischen Lecithinen isolierten Phospholipide, wobei insbesondere in der erfindungsgemäßen Zubereitung 1,2-Diacylglycero-3-Phosphocholin[(3-sn-Phosphatidyl)cholin] in Mischung mit anderen Phospholipiden enthalten ist. Als weitere Phospholipide, die in der erfindungsgemäßen Zusammensetzung enthalten sein können, sind vorzugsweise 1,2-Diacylglycero-3-Phosphoethanolamin, 1,2-Diacylglycero-3-Phosphoinositol, 1,2-Diacylglycero-3-Phosphoserin, 1,2-Diacylglycero-3-Phosphoglycerol und 1,2-Diacylglycero-3-Phosphat jeweils allein oder in Mischung zu nennen.

Weist die erfindungsgemäße Zusammensetzung die zuvor genannten und mit Linolsäure acylierten Phospholipide auf, so ist zwischen der Acylierung mit Linolsäure in 1-Stellung, der Acylierung mit Linolsäure in 1- und 2-Stellung sowie der Acylierung mit Linolsäure in 2-Stellung zu unterscheiden. Dementsprechend weist die erfindungsgemäße Zubereitung 1-Linolylglycero-3-Phosphate, 1,2-Dilinolylglycero-3-Phosphate und/oder 2-Linolylglycero-3-Phosphate der vorstehend genannten Art auf.

Eine besonders geeignete Ausführungsform der erfindungsgemäßen Zubereitung enthält mindestens ein mit Linolsäure acyliertes Phospholipid bzw. Phospholipidgemisch der vorstehend genannten Art, wobei mindestens 60 Gew.% der Acylreste Linolsäure darstellen.

Bei einer besonders geeigneten und sehr wirksamen Weiterbildung der zuvor beschriebenen erfindungsgemäßen Zubereitung enthält die Zubereitung ein solches acyliertes Phospholipidgemisch, bei dem die in dem Gemisch enthaltenen Acylreste zu
61 - 73 Gew.% aus dem Linolsäurerest,
10 - 14 Gew.% aus dem Palmitinsäurerest,
8 - 12 Gew.% aus dem Ölsäurerest,
4 - 6 Gew.% aus dem Linolensäurerest,
3 - 5 Gew.% aus dem Stearinsäurerest und
bis zu 2 Gew.% aus anderen Fettsäureresten
bestehen.

Insbesondere dann, wenn die zuvor beschriebenen vorteilhafte Weiterbildung der erfindungsgemäßen pharmazeutischen Zubereitung 15 - 30 Gew.%, bezogen auf die anwendungsfertige Zubereitung, eines Phospholipidgemisches, mit einem Gehalt von 70 bis 100 Gew.% (bezogen auf das Phospholipidgemisch) 1,2-Diacylglycero-3-Phosphocholin beinhaltet, wobei das in dem Phospholipidgemisch enthaltene Acylrestegemisch mindestens 60 Gew.% Linolsäurereste enthält, weist eine derartige Ausführungsform der erfindungsgemäßen Zubereitung hervorragende pharmazeutische Wirkungen in bezug auf die Behandlung von Akne und der damit verbundenen Begleiterkrankungen auf. Die dann noch verbleibenden maximal 40 Gew.% Acylreste umfassen insbesondere den Palmitinsäurerest, den Ölsäurerest, den Linolsäurerest und/oder den Stearinsäurerest, vorzugsweise in den zuvor angegebenen Massenverhältnissen.

Wie bereits vorstehend erwähnt ist, kann das in der erfindungsgemäßen Zubereitung vorgesehene Phospholipid ein Phospholipidgemisch sein. Hierfür kommen insbesondere die bereits vorstehend genannten 1,2-Diacylglycero-3-phosphat (1,2-Diacylglycero-3-phosphoethanolamin, 1,2-Diacylglycero-3-phosphoinositol, 1,2-Diacylglycero-3-phosphoserin, 1,2-Diacylglycero-3-phosphoglycerol und/oder 1,2-Diacylglycero-3-phosphat) in Frage, wobei vorzugsweise bis zu 30 Gew.% der vorstehend genannten 1,2-Diacylglycero-3-phosphate in dem Phospholipidgemisch enthalten sind, während eine derartige Ausführungsform der erfindungsgemäßen Zubereitung dann mindestens 70 Gew.% des 1,2-Diacylglycero-3-phosphocholin aufweist. Hierbei beziehen sich die zuvor genannten prozentualen Massenangaben auf die Gesamtmasse des in der erfindungsgemäßen Zubereitung enthaltenen Phospholipidgemisches, das, wie bereits vorstehend ausgeführt ist, vorzugsweise in der anwendungsfertigen Zubereitung zu 5 bis 50 Gew.%, bezogen auf die Masse der fertigen Zubereitung, enthalten ist.

Eine andere, ebenfalls besonders geeignete Ausführungsform der erfindungsgemäßen Zubereitung beinhaltet als Phospholipid ein 1,2-Diacylglycero-3-phosphocholin, bei dem der 1-Acylrest
45 - 61 Gew.% Linolsäurereste,
19 - 26 Gew.% Palmitinsäurerest,
8 - 12 Gew.% Ölsäurereste,
4 - 6 Gew.% Linolensäurereste,
6 - 9 Gew.% Stearinsäurereste und/oder
2 Gew.% andere Fettsäurereste
umfaßt. Um hierbei sicherzustellen, daß in einer derartigen Zubereitung auch die erforderliche Menge an Linolsäure enthalten ist, kann entweder freie Linolsäure zugesetzt werden oder der in 2-Stellung befindliche Acylrest entsprechend mit Linolsäure acyliert sein, wobei vorzugsweise solche 1,2-Diacylglycero-3-phosphocholine eingesetzt werden, bei denen der 1-Acylrest die vorstehend angegebene Zusammensetzung aufweist und der 2-Acylrest
77 - 85 Gew.% Linolsäurereste,
1 - 2 Gew.% Palmitinsäurereste,
8 - 12 Gew.% Ölsäurereste,
4 - 6 Gew.% Linolensäurereste,
0 - 1 Gew.% Stearinsäurereste und/oder 2 Gew.% andere Fettsäurereste
umfaßt.

Bezüglich der Konzentration an mit Linolsäure acyliertem Trägermaterial das in der erfindungsgemäßen Zubereitung als Wirkstoff enthalten ist, ist festzuhalten, daß diese Konzentration zwischen 1 Gew.% und 30 Gew.%, vorzugsweise zwischen 3 Gew.% und 18 Gew.%, variiert. Wird die erfindungsgemäße Zubereitung als kosmetische Zubereitung eingesetzt, weist eine derartige kosmetische Zubereitung den Wirkstoff in Konzentrationen auf, die vorzugsweise zwischen 1 Gew.% und 8 Gew.% variieren, während entsprechende pharmazeutische Zubereitungen Wirkstoffkonzentrationen enthalten, die insbesondere zwischen 15 Gew.% und 30 Gew.% liegen. Hierbei beziehen sich die zuvor wiedergegebenen prozentualen Massenangaben auf die anwendungsfertige Zubereitung.

Wie bereits vorstehend ausgeführt ist, besitzt die erfindungsgemäße Zubereitung vorzugsweise eine gelartige oder flüssige Konsistenz. Dies bedeutet, daß die erfindungsgemäße Zubereitung insbesondere als Gel, Lösung, Lotion, Salbe, oder Creme formuliert wird. Hierbei können übliche Hilfsstoffe, wie beispielsweise Konsistenzgeber (CMC, Gabapol, Alginate, Xantan) und/oder dermatologische Konservierungsmittel zugesetzt werden, wobei jedoch darauf zu achten ist, daß Stoffe, die die Fettproduktion anregen, die die Haut abdecken oder die selbstfettend wirken, vermieden werden sollen. Darüber hinaus sollte das Konservierungsmittel selbst nicht penetrieren, wobei als bevorzugte Konservierungsstofffe somit wasserlösliche Alkohole, insbesondere Propylenglykol, in der erfindungsgemäßen Zubereitung enthalten ist.

Um die notwendige Sterilität bei solchen flüssigen Formulierungen der erfindungsgemäßen Zubereitung, die keine organischen Lösungsmittel und insbesondere auch keine Alkohole enthalten, sicherzustellen, sieht eine Weiterbildung dieser Ausführungsform vor, daß die flüssige Zubereitung in gasdichten Ampullen abgepackt ist. Hierbei weist dann die gasdichte Ampulle so viel von der flüssigen erfindungsgemäßen Zubereitung auf, die ausreicht, um die erfindungsgemäße Zubereitung einmalig anzuwenden.

Enthält die erfindungsgemäße Zubereitung einen Elektrolyten, so kann als Elektrolyt jeder, im medizinischer Bereich eingesetzter Elektrolyt eingesetzt werden, vorzugsweise jedoch handelt es sich bei dem Elektrolyten um eine physiologische Kochsalzlösung. Die zweite Komponente, die, wie bereits vorstehend erwähnt ist, von der ersten Komponente getrennt verpackt ist, enthält bei dieser Ausführungsform das mit Linolsäure acylierte Trägermaterial sowie ggf. ein Lösungsmittel der vorstehend genannten Art. Der Zusatz des Lösungsmittels zur zweiten Komponente weist den Vorteil auf, daß dann bei einer derartigen Abwandlung der erfindungsgemäßen Zubereitung zwei flüssige Komponenten vorliegen, die unmittelbar vor der Anwendung sehr leicht miteinander vermischt werden können.

Um bei der erfindungsgemäßen Zubereitung, die zwei Komponenten vorsieht, den erforderlichen pH-Wert einzustellen, ist bei einer Weiterbildung eine der beiden Komponenten, insbesondere die erste Komponente, mit einem geeigneten pH-Regulator versehen. Hierbei handelt es sich dann vorzugsweise um ein wäßriges Puffersystem oder um eine entsprechende Lauge, wobei die pH-Werte der anwendungsfertigen und vermischten Zubereitung zwischen 5,5 und 8, vorzugsweise zwischen 6,5 und 7,5, variieren.

Bezüglich der Konzentrationen der Bestandteile bei der erfindungsgemäßen Zubereitung, die die erste und die zweite, unmittelbar vor der Anwendung zu vermischenden Komponenten aufweist, ist festzuhalten, daß hierbei insbesondere die erste Komponente zwischen
50 Gew.% bis 80 Gew.% Wasser sowie zwischen 0 Gew.% und 20 Gew.% Lösungsmittel
und die zweite Komponente zwischen
3 Gew.% und 15 Gew.% des mit Linolsäure acylierten phospholipidischen Trägermaterials sowie 0 Gew.% bis 15 Gew.% Lösungsmittel
aufweist.

Eine Weiterbildung dieser zuvor beschriebenen Ausführungsform sieht vor, daß das in der ersten Komponente enthaltene Wasser (50 Gew.% bis 80 Gew.%) durch eine wäßrige Elektrolytlösung und vorzugsweise durch eine wäßrige physiologische Kochsalzlösung (0,5 Gew.% bis 2,5 Gew.% Kochsalz) vollständig oder teilweise ersetzt ist. Eine derartige, elektrolythaltige Weiterbildung zeichnet sich durch eine hohe Sterilität und eine besonders lange Haltbarkeit auf.

Desweiteren können in der ersten Komponente und/oder der zweiten Komponente noch übliche Hilfsstoffe, wie beispielsweise pH-Regulatoren, Puffersysteme, Emulgatoren und/oder Verdickungsmittel, enthalten sein.

Wie bereits vorstehend wiederholt ausgeführt ist, wird die erfindungsgemäße Zubereitung insbesondere zur Prophylaxe und/oder Therapie von Akne und/oder den mit Akne verbundenen Begleiterkrankungen der Haut verwendet. Hier konnte überraschend festgestellt werden, daß die erfindungsgemäße Zubereitung bei Akne und/oder den mit Akne verbundenen Begleiterkrankungen der Haut sehr schnell zu einer vollständigen Heilung dieser Erkrankungen führte, ohne daß dabei unangenehme oder störende Nebenwirkungen auftraten.

Unter den Begriff Akne im Sinne der vorliegenden Beschreibung fallen alle Erkrankungen der Haut, bei denen eine Ausbildung von Komedonen, Papeln, Pusteln oder Abszessen erfolgt. Insbesondere fallen hier runter Acne cachecticorum, Acne necroticans, Acne varioliformis, Acne picea, Acne vulgaris, Acne conglobata sowie Acne juvenilis.

Die erfindungsgemäße Zubereitung kann zusätzlich noch gängige Therapeutika zur topischen Applikation, wie insbesondere Erythromycin, seine Derivate und/oder entsprechende Salze, Tetracyclin-HCl und/oder Retinolsäure (Tretinoin USP xx 1), enthalten.

Geeignete Mengenverhältnisse der zuvor genannten gängigen Therapeutika sind für
Erythromycin 0,5 - 4 Gew.%,
Tetracyclin-HCl 1 - 5 Gew.%,
Azelainsäure 5 - 20 Gew.% und
Tretinoin 0,025 - 0,1 Gew.%.

Vorteilhafte Weiterbildungen der erfindungsgemäßen Zubereitung sind in den Unteransprüchen angegeben.

Die erfindungsgemäße Zubereitung wird nachfolgend anhand von Ausführungsbeispielen näher erläutert.

Für die Herstellung der Zusammensetzungen gemäß der Ausführungsbeispiele 1 - 5 wurde ein Soja-Phospholipid A verwendet, wobei das Phospholipid A als Hauptbestandteil 76 ± 3 Gew.% Phosphatidylcholin sowie 3 ± 3 Gew.% Lysophosphatidylcholin enthielt.

### Ausführungsbeispiel 1

Es wurden 2 Systeme jeweils getrennt hergestellt, die erst unmittelbar direkt vor der ersten Anwendung miteinander vermischt werden.

Hierbei enthielt das 1. System
- 1,2: Gewichtsteile Erythromycin und
- 20: Gewichtsteile Ethanol,
während das 2. System aus
- 5: Gewichtsteile Phospholipid A,
- 16: Gewichtsteile Propylenglykol und
- 57,8: Gewichtsteile Wasser
bestanden.

Die zuvor genannten beiden System wurden erst unmittelbar vor ihrer Anwendung zusammengegeben und vermischt, wobei eine gebrauchsfertige, liposomale Dispersion entstand, die zum baldigen Verbrauch bestimmt war.

### Ausführungsbeispiel 2

Es wurde eine Zusammensetzung unter Verwendung des eingangs genannten Soja-Phospholipids A hergestellt, wobei die Zusammensetzung eine erste, im Behälter 1 verpackte Komponente sowie eine zweite, im Behälter 2 verpackte Komponente aufwies.

Der Behälter 1 wies die folgenden Inhaltsstoffe auf:

| | |
|---|---|
| demineralisiertes Wasser | 73,83 g |
| Ethanol DAB 9 | 10 g |
| 10 %ige NaOH | 0,17 g |

Der Behälter 2 beinhaltete folgende Inhaltsstoffe:

| | |
|---|---|
| Phospholipid A | 10 g |
| Ethanol DAB 9 | 6 g |

### Ausführungsbeispiel 3

Es wurde ebenfalls eine auf zwei Behälter aufgeteilte Zusammensetzung hergestellt. Hierbei wies der Behälter 1

| | |
|---|---|
| demineralisiertes Wasser | 73,83 g |
| Isopropylalkohol | 10 g |
| Natriumhydroxid, 10 %ig | 0,17 g |

und
der Behälter 2

| | |
|---|---|
| Phospholipid A | 10 g |
| Isopropylalkohol | 6 g |

auf.

### Ausführungsbeispiel 4

Es wurde eine weitere Zusammensetzung hergestellt, wobei im Behälter 1

| | |
|---|---|
| physiologische Kochsalzlösung (1 %ig) | 73,83 g |
| Ethanol DAB 9 | 10 g |
| Natriumhydroxid 10 %ig | 0,17 g |

waren und sich im Behälter 2

| | |
|---|---|
| Phospholipid A | 10 g |
| Ethanol DAB 9 | 6 g |

befanden.

### Ausführungsbeispiel 5

Wie bei den vorstehend genannten Ausführungsbeispielen 2 bis 4 wurde eine aus zwei Komponenten bestehende Zusammensetzung erstellt, wobei der erste Behälter 1

| | |
|---|---|
| physiologische Kochsalzlösung (1 %ig) | 73,83 g |
| Isopropylalkohol | 10 g |
| Natriumhydroxidlösung (10 %ig) | 0,17 g |

und der zweite Behälter

| | |
|---|---|
| Phospholipid A | 10 g sowie |
| Isopropylalkohol | 6 g |

aufwies.

Die zuvor in den Ausführungsbeispielen 2 bis 5 in separate Behältern abgefüllten Bestandteile der Zusammensetzung wurden durch kurzes Schütteln (30 Sekunden) miteinander vermischt. Hierbei entstand aus den Bestandteilen des Ausführungsbeispiels 2 ein bräunliches, liposomales Gel mit einem mittleren Liposomendurchmesser von 380 nm und einen pH-Wert von 6,8, aus den Bestandteilen des Ausführungsbeispiel 3 ebenfalls ein bräunliches, liposomales Gel mit einer mittleren Teilchengröße von 311 nm und einem pH-Wert von 7,0, aus den Bestandteilen des Ausführungsbeispiels 4 eine milchige, liposomale Flüssigkeit mit einer mittleren Teilchengröße von 540 nm und einem pH-Wert von 6,6 und aus den Bestandteilen des Ausführungsbeispiels 5 eine gelblich flüssige liposomale Zubereitung mit einer mittleren Liposomenteilchengröße von 281 nm und einem pH-Wert von 6,8.

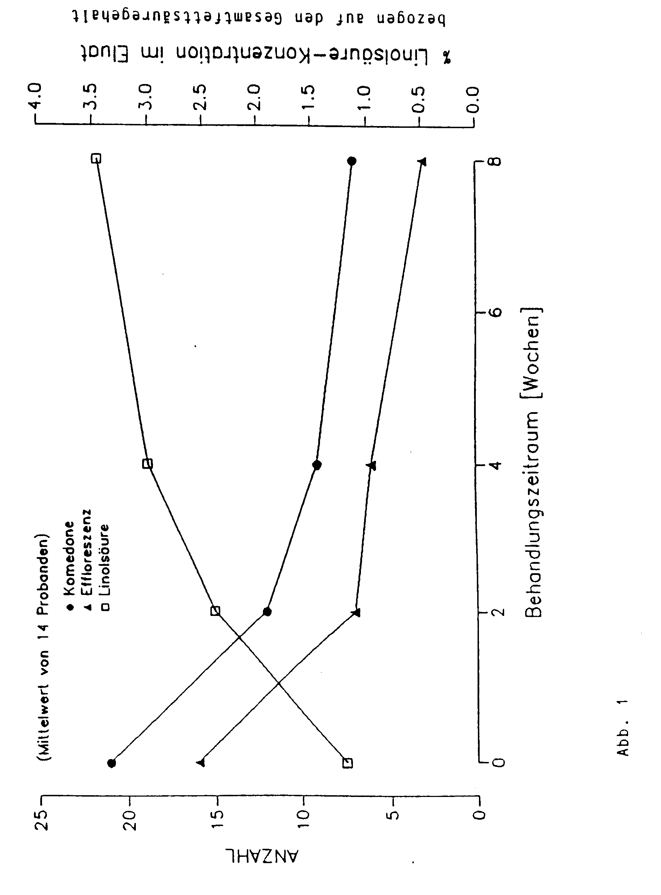

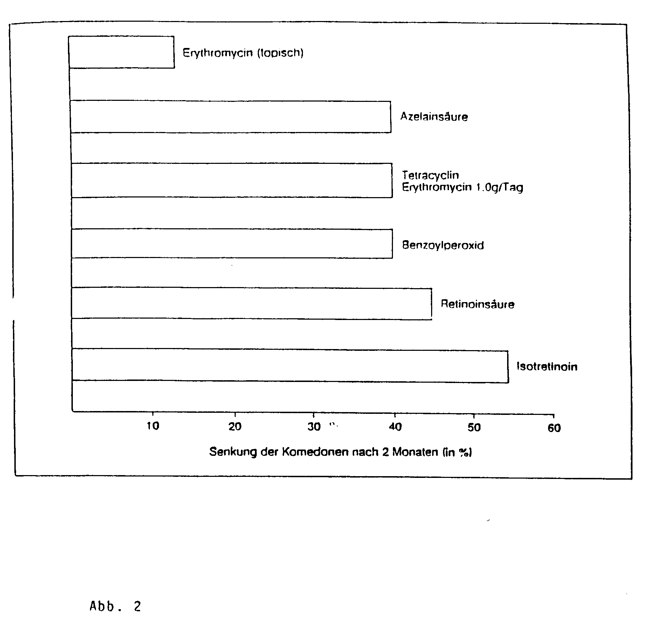

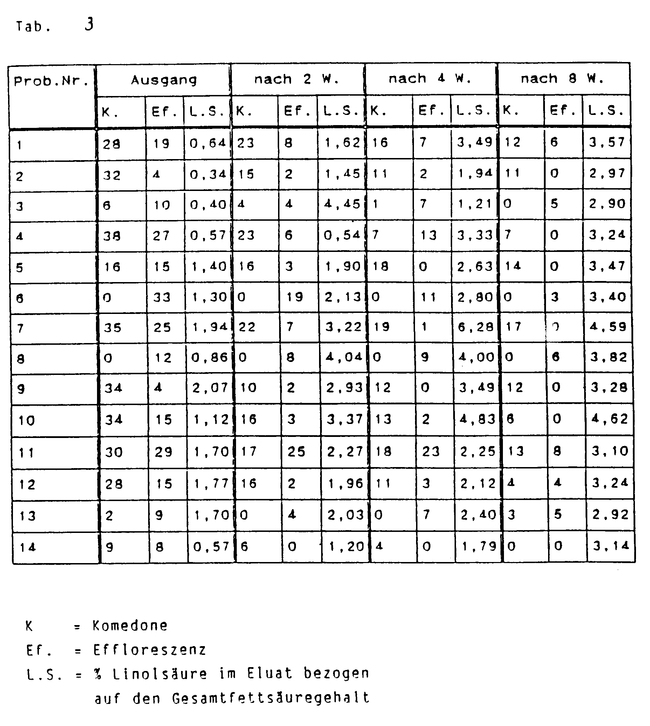

## Patentansprüche

1. Pharmazeutische und/oder kosmetische Zubereitung zur topischen Anwendung zur Prophylaxe und/oder Therapie von Akne und/oder der mit Akne verbundenen Begleiterkrankungen der Haut und/oder unreiner Haut, enthaltend mindestens ein phospholipidisches Trägermaterial sowie mindestens einen Wirkstoff, wobei die gelartige oder flüssige Zubereitung einen solchen Wirkstoff aufweist, der chemisch derart an das phospholipidische Trägermaterial gebunden ist, daß das mit Linolsäure acylierte phospholipidische Trägermaterial ein 1,2-Diacylglycero-3-Phosphatidylcholin ist, dadurch gekennzeichnet, daß die Zubereitung eine erste Komponente und eine hiervon getrennt verpackte zweite Komponente umfaßt, wobei die erste Komponente
ein Lösungsmittel,
mindestens einen Elektrolyten und/oder
Wasser,
und die zweite Komponente
das mit Linolsäure acylierte phospholipidische Trägermaterial
sowie
ggf. Lösungsmittel
enthält, wobei die beiden Komponenten zur Erstellung einer anwendungsfertigen Zubereitung miteinander mischbar sind.

2. Zubereitung nach Anspruch 1, dadurch gekennzeichnet, daß die Zubereitung desweiteren noch ein mit Linolsäure acyliertes Phospholipid oder Phospholipidgemisch enthält.

3. Zubereitung nach Anspruch 1 oder 2, dadurch gekennzeichnet, daß sie ein mit Linolsäure acyliertes Phospholipid enthält, wobei mindestens 60 Gew.% des Acylrestes Linolsäure sind.

4. Zubereitung nach einem der vorangehenden Ansprüche, dadurch gekennzeichnet, daß die Zubereitung ein solches acyliertes Phospholipidgemisch enthält, bei dem die in dem Gemisch enthaltenen Acylreste zu
61 - 73 Gew.% aus dem Linolsäurerest,
10 - 14 Gew.% aus dem Palmitinsäurerest,
8 - 12 Gew.% aus dem Ölsäurerest,
4 - 6 Gew.% aus dem Linolensäurerest,
3 - 5 Gew.% aus dem Stearinsäurerest und/oder
2 Gew.% aus anderen Fettsäureresten
bestehen.

5. Zubereitung nach einem der vorangehenden Ansprüche, dadurch gekennzeichnet, daß das Phospholipidgemisch eines oder mehrerer anderer 1,2-Diacylglycero-3-phosphate, ausgewählt aus der Gruppe, bestehend aus
1,2-Diacylglycero-3-phosphoethanolamin,
1,2-Diacylglycero-3-phosphoinositol,
1,2-Diacylglycero-3-phosphoserin,
1,2-Diacylglycero-3-phosphoglycerol und
1,2-Diacylglycero-3-phosphat
enthält.

6. Zubereitung nach einem der vorangehenden Ansprüche, dadurch gekennzeichnet, daß in dem 1,2-Diacylglycero-3-phosphocholin der 1-Acylrest
45 - 61 Gew.% Linolsäurereste,
19 - 26 Gew.% Palmitinsäurereste,
8 - 12 Gew.% Ölsäurereste,
4 - 6 Gew.% Linolensäurereste,
6 - 9 Gew.% Stearinsäurereste und/oder
2 Gew.% andere Fettsäurereste
enthält.

7. Zubereitung nach einem der vorangehenden Ansprüche, dadurch gekennzeichnet, daß in dem 1,2-Diacylglycero-3-phosphocholin der 2-Acylrest
77 - 85 Gew.% Linolsäurereste,
1 - 2 Gew.% Palmitinsäurereste,
8 - 12 Gew.% Ölsäurereste,
4 - 6 Gew.% Linolensäurereste,
0 - 1 Gew.% Stearinsäurereste und/oder
2 Gew.% andere Fettsäurereste
enthält.

8. Zubereitung nach einem der vorangehenden Ansprüche, dadurch gekennzeichnet, daß die Zubereitung einen wasserlöslichen Alkohol und/oder Wasser enthält.

9. Zubereitung nach Anspruch 8, dadurch gekennzeichnet, daß sie als Lösungsmittel Wasser aufweist und in gasdichten Ampullen abgepackt ist.

10. Zubereitung nach einem der vorangehenden Ansprüche, dadurch gekennzeichnet, daß eine der beiden Komponenten, vorzugsweise die erste Komponente, noch einen pH-Regulator umfaßt.

11. Zubereitung nach einem der vorangehenden Ansprüche, dadurch gekennzeichnet, daß die erste und/oder die zweite Komponente noch übliche Hilfsstoffe aufweisen.

12. Zubereitung nach einem der vorangehenden Ansprüche, dadurch gekennzeichnet, daß die erste Komponente
50 Gew.% bis 80 Gew.% Wasser sowie 0 Gew.% bis 20 Gew.% Lösungsmittel,
und die zweite Komponente
3 Gew.% bis 15 Gew.% des mit Linolsäure acylierten phospholipidischen Trägermaterials sowie 0 Gew.% bis 15 Gew.% Lösungsmittel
enthält.

13. Zubereitung nach Anspruch 12, durch gekennzeichnet, daß das Wasser in der ersten Komponente eine wäßrige physiologische Kochsalzlösung ist.

14. Zubereitung nach einem der vorangehenden Ansprüche, dadurch gekennzeichnet, daß die Zubereitung einen weiteren Wirkstoff, ausgewählt aus der Gruppe Erythromycin, ein Erythromycinsalz, ein Erythromycinderivat, Tetracyclin, Azelainsäure und/oder Retinolsäure enthält.

15. Zubereitung nach einem der vorangehenden Ansprüche, dadurch gekennzeichnet, daß das mit Linolsäure acylierte Phospholipid ein pflanzliches Phospholipid, insbesondere ein Sonnenblumen- oder Soja-Phospholipid, ist.

## Claims

1. A pharmaceutical and/or cosmetic composition for the topical use for the prophylaxis and/or the therapy of acne and/or accompanying disorders of the skin related with acne and/or impure skin, comprising at least one phospholipidic carrier material as well as at least one active ingredient, whereby the gel-like or liquid composition contains such an active ingredient which is chemically bonded to the phospholipidic carrier material in such a way that the phospholipidic carrier material being acylated with linoleic acid is a 1,2-diacylglycero-3-phosphatidylcholine, characterised in that the composition comprises a first component and a second component packed separately from each other, whereby the first component contains
a solvent,
at least one electrolyte and/or
water,
and the second component contains
the phospholipidic carrier material being acylated with linoleic acid as well as optionally a solvent,
whereby the two components are mixed with each other for the manufacturing of a ready-to-apply composition.

2. The composition according to claim 1, characterised in that the composition additionally comprises a phospholipid or a phospholipid mixture being acylated with linoleic acid.

3. The composition according to claim 1 or 2, characterised in that the composition comprises a phospholipid being acylated with linoleic acid, whereby at least 60 % by weight of the acyl group are linoleic acid.

4. The composition according to one of the preceding claims, characterised in that the composition comprises such an acylated phospholipid mixture having acyl groups, containing in the mixture, of
61 - 73 % by weight linoleic acid group,
10 - 14 % by weight palmitic acid group,
8 - 12 % by weight oleic acid group,
4 - 6 % by weight linolenic acid group,
3 - 5 % by weight stearic acid group and/or
2 % by weight other fatty acid groups.

5. The composition according to one of the preceding claims, characterised in that the phospholipid mixture comprises one or more other 1,2-diacylglycero-3-phosphates selected from the group consisting of
1,2-diacylglycero-3-phosphoethanolamine,
1,2-diacylglycero-3-phosphoinositol,
1,2-diacylglycero-3-phosphoserine,
1,2-diacylglycero-3-phosphoglycerol and
1,2-diacylglycero-3-phosphate.

6. The composition according to one of the preceding claims, characterised in that the 1-acyl group of the 1,2-diacylglycero-3-phosphocholine contains
45 - 61 % by weight linoleic acid groups,
19 - 26 % by weight palmitic acid groups,
8 - 12 % by weight oleic acid groups,
4 - 6 % by weight linolenic acid groups,
6 - 9 % by weight stearic acid groups and/or
2 % by weight other fatty acid groups.

7. The composition according to one of the preceding claims, characterised in that the 2-acyl group of the 1,2-diacylglycero-3-phosphocholine contains
77 - 85 % by weight linoleic acid groups,
1 - 2 % by weight palmitic acid groups,
8 - 12 % by weight oleic acid groups,
4 - 6 % by weight linolenic acid groups,
0 - 1 % by weight stearic acid groups and/or
2 % by weight other fatty acid groups.

8. The composition according to one of the preceding claims, characterised in that the composition comprises a water-soluble alcohol and/or water.

9. The composition according to claim 8, characterised in that the composition contains water as solvent and that the composition is filled in gas-tight ampoules.

10. The composition according to one of the preceding claims, characterised in that one of the two components, preferably the first component, additionally comprises a pH-regulator.

11. The composition according to one of the preceding claims, characterised in that the first and/or the second component additionally comprise common auxiliary agents.

12. The composition according to one of the preceding claims, characterised in that the first component comprises
50 % by weight to 80 % by weight water as well as 0 % by weight to 20 % by weight solvents,
and that the second component comprises
3 % by weight to 15 % by weight of the phospholipidic carrier being material acylated with linoleic acid as well as 0 % by weight to 15 % by weight solvents.

13. The composition according to claim 12, characterised in that the water in the first component is an aqueous physiological sodium chloride solution.

14. The composition according to one of the preceding claims, characterised in that the composition comprises a further active ingredient selected from the group consisting of erythromycin, a salt of erythromycin, a erythromycin derivative, tetracycline, azelaic acid and/or retinoic acid.

15. The composition according to one of the preceding claims, characterised in that the phospholipid being acylated with linoleic acid is a phospholipid of plant origin, particularly a sunflower phospholipid or a soybean phospholipid.

## Revendications

1. Préparation pharmaceutique et/ou cosmétique à appliquer localement pour la prophylaxie et/ou la thérapie d'acné et/ou des maladies secondaires de la peau associées à de l'acné et/ou de peau souillée, contenant au moins une matière de support phospholipidique ainsi qu'au moins une substance active, la préparation de type gel ou liquide présentant une substance active qui est fixée chimiquement sur la matière de support phospholipidique de telle façon que la matière de support phospholipidique acylée par de l'acide linoléique soit une 1,2-diacylglycéro-3-phosphatidylcholine, caractérisée en ce que la préparation comporte un premier composant et un deuxième composant emballé de manière séparée du premier, le premier composant contenant
un solvant,
au moins un électrolyte et/ou
de l'eau,
et le deuxième composant
la matière de support phospholipidique acylée par de l'acide linoléique,
ainsi qu'éventuellement
du solvant,
les deux composants étant miscibles l'un à l'autre pour la réalisation d'une préparation prête à l'usage.

2. Préparation suivant la revendication 1, caractérisée en ce que la préparation contient encore un phospholipide ou mélange de phospholipides acylé par de l'acide linoléique.

3. Préparation suivant l'une des revendications 1 et 2, caractérisée en ce qu'elle contient un phospholipide acylé par de l'acide linoléique, dans lequel au moins 60% en poids du radical acyle sont de l'acide linoléique.

4. Préparation suivant l'une des revendications précédentes, caractérisée en ce que la préparation contient un mélange de phospholipides acylé dans lequel les radicaux acyle contenus dans le mélange sont constitués de
61-73% en poids du radical d'acide linoléique,
10-14% en poids du radical d'acide palmitique,
8-12% en poids du radical d'acide oléique,
4- 6% en poids du radical d'acide linolénique,
3- 5% en poids du radical d'acide stéarique, et/ou
2% en poids d'autres radicaux d'acides gras.

5. Préparation suivant l'une des revendications précédentes, caractérisée en ce que le mélange de phospholipides contient un ou plusieurs autres 1,2-diacylglycéro-3-phosphates, choisis parmi le groupe comprenant
de la 1,2-diacylglycéro-3-phosphoéthanolamine,
du 1,2-diacylglycéro-3-phosphoinositol,
de la 1,2-diacylglycéro-3-phosphosérine,
du 1,2-diacylglycéro-3-phosphoglycérol et
du 1,2-diacylglycéro-3-phosphate.

6. Préparation suivant l'une des revendications précédentes, caractérisée en ce que, dans la 1,2-diacylglycéro-3-phosphocholine, le radical 1-acyle contient
45-61% en poids de radicaux d'acide linoléique,
19-26% en poids de radicaux d'acide palmitique,
8-12% en poids de radicaux d'acide oléique,
4- 6% en poids de radicaux d'acide linolénique,
6- 9% en poids de radicaux d'acide stéarique, et/ou
2% en poids d'autres radicaux d'acides gras.

7. Préparation suivant l'une des revendications précédentes, caractérisée en ce que, dans la 1,2-diacylglycéro-3-phosphocholine, le radical 2-acyle contient
77-85% en poids de radicaux d'acide linolénique,
1- 2% en poids de radicaux d'acide palmitique,
8-12% en poids de radicaux d'acide oléique,
4- 6% en poids de radicaux d'acide linolénique,
0- 1% en poids de radicaux d'acide stéarique, et/ou
2% en poids d'autres radicaux d'acides gras.

8. Préparation suivant l'une des revendications précédentes, caractérisée en ce que la préparation contient un alcool soluble dans l'eau et/ou de l'eau.

9. Préparation suivant la revendication 8, caractérisée en ce que, comme solvant, elle présente de l'eau et en ce qu'elle est emballée dans des ampoules étanches aux gaz.

10. Préparation suivant l'une des revendications précédentes, caractérisée en ce qu'un des deux composants, de préférence le premier composant, comporte encore un régulateur de pH.

11. Préparation suivant l'une des revendications précédentes, caractérisée en ce que le premier composant et/ou le deuxième présentent encore des adjuvants usuels.

12. Préparation suivant l'une des revendications précédentes, caractérisée en ce que le premier composant contient
50% en poids à 80% en poids d'eau, ainsi que 0% en poids à 20% en poids de solvant,
et le deuxième composant
3% en poids à 15% en poids de la matière de support phospholipidique acylée par de l'acide linoléique, ainsi que
0% en poids à 15% en poids de solvant.

13. Préparation suivant la revendication 12, caractérisée en ce que l'eau dans le premier composant est une solution saline physiologique aqueuse.

14. Préparation suivant l'une des revendications précédentes, caractérisée en ce que la préparation contient une autre substance active choisie parmi le groupe comprenant de l'érythromycine, un sel d'érythromycine, un dérivé d'érythromycine, de la tétracycline, de l'acide azélaïque et/ou de l'acide rétinoïque.

15. Préparation suivant l'une des revendications précédentes, caractérisée en ce que le phospholipide acylé par de l'acide linoléique est un phospholipide végétal, en particulier un phospholipide de tournesol ou de soja.
